# EUROPÄISCHE PATENTSCHRIFT

(11) **EP 2 147 688 B1**
(45) Veröffentlichungstag und Bekanntmachung des Hinweises auf die Patenterteilung: **20.07.2016**
(21) Anmeldenummer: 09163889.0
(22) Anmeldetag: 26.06.2009
(51) Int. Cl.: A61L 31/02, A61L 31/10, A61L 31/14

(54) **Endoprothese und Verfahren zur Herstellung derselben**
Endoprosthesis and method for manufacturing the same
Endoprothèse et son procédé de fabrication

(30) Priorität: 23.07.2008 DE 102008040640
(43) Veröffentlichungstag der Anmeldung: 27.01.2010
(73) Patentinhaber: Biotronik VI Patent AG, 6341 Baar (CH)
(72) Erfinder: Klocke, Björn, 8006, Zürich (CH)
(74) Vertreter: Galander, Marcus

(56) Entgegenhaltungen:
- EP-A2- 2 018 882
- WO-A1-2005/065576
- WO-A2-2008/034474

## Beschreibung

Die Erfindung betrifft eine Endoprothese oder Implantat, insbesondere eine intraluminale Endoprothese, zum Beispiel einen Stent, mit einem Grundgitter bestehend aus einem im Wesentlichen biodegradierbaren Material und einer auf dem biodegradierbaren Material angeordneten Beschichtung sowie ein Verfahren zur Herstellung einer derartigen Endoprothese.

Stents sind endovaskuläre Prothesen, die zur Behandlung von Stenosen (Gefäßverengungen) eingesetzt werden können. Sie weisen ein rohrförmiges oder hohlzylinderförmiges Grundgitter auf, das an beiden Längsenden offen ist. Das rohrförmige Grundgitter aus dem Grundmaterial einer derartigen Endoprothese wird in das zu behandelnde Gefäß eingesetzt und dient dazu, das Gefäß zu stützen.

Derartige Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatzpunkt zur Lösung dieses Problems besteht darin, den Stent aus einem biodegradierbaren Werkstoff zu fertigen.

Unter Biodegradation werden hydrolytische, enzymatische und andere stoffwechselbedingte chemische Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für das Grundgitter biodegradierbarer Endoprothesen geeignete Werkstoffe (Grundmaterial) können beispielsweise polymerer oder metallischer Natur sein. Das Grundgitter kann auch aus mehreren Werkstoffen bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium, Eisen, Zink und/oder Wolfram. Die vorliegende Erfindung bezieht sich vorzugsweise auf Stents oder andere Endoprothesen, deren biodegradierbarer Werkstoff Magnesium oder eine Magnesiumlegierung, besonders bevorzugt die Legierung WE43, und/oder ein biodegradierbares Polymer, besonders bevorzugt PLLA, enthält. Diese Stoffe weisen als Stentgrundmaterial eine besonders geeignete Kombination mechanischer, biologischer und korrosiver Eigenschaften auf.

Es sind bereits Stents bekannt, die Beschichtungen mit verschiedenen Funktionen aufweisen. Bei der Realisierung biodegradierbarer Implantate besteht das Problem, die Degradierbarkeit entsprechend der angestrebten Therapie zu steuern. Es konnte bisher noch kein Stent gebaut werden, der im für viele therapeutische Anwendungen wichtigen Zielkorridor von vier Wochen bis sechs Monaten seine Integrität verliert. Hierbei wird unter Integrität, d. h. mechanischer Integrität, die Eigenschaft verstanden, dass der Stent bzw. die Endoprothese gegenüber dem undegradierten Stent kaum mechanische Einbußen besitzt. Dies bedeutet, dass der Stent mechanisch noch so stabil ist, dass der Kollapsdruck lediglich geringfügig, d. h. höchstens auf 80% des Nominalwerts, abgefallen ist. Der Stent kann somit bei vorhandener Integrität seiner Hauptfunktion, dem Aufhalten des Gefäßes, noch nachkommen. Alternativ kann die Integrität dadurch definiert werden, dass der Stent mechanisch so stabil ist, dass er in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers, oder kaum angebrochene tragende Streben aufweist.

Für den genannten Zielkorridor der Degradation besonders vielversprechend haben sich degradierbare Magnesium-Stents erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass bei Magnesium-Stents der Kollapsdruck über die Zeit zu schnell verringert wird und/oder die Verringerung des Kollapsdrucks eine zu große Variabilität aufweist und damit zu unbestimmbar ist.

Grundsätzlich existieren drei bekannte Ansätze, um den gewünschten zeitlichen Zielkorridor des Integritätsverlustes zu einzustellen. Erstens kann ein dickeres, optimiertes Stentdesign gewählt werden. Zweitens kann eine optimierte, langsam degradierende Magnesiumlegierung für den Stent verwendet werden oder drittens können Oberflächenschichten vorgesehen werden, die den Degradationsangriff des Magnesium-Grundgitters verlangsamen bzw. beschleunigen und/oder den Zeitpunkt des Einsetzens der Degradation beeinflussen. Die Möglichkeit, das Degradationsverhalten entsprechend der ersten oder zweiten Lösungsmöglichkeit zu variieren, ist stark eingeschränkt und reicht möglicherweise nicht für eine ökonomisch und klinisch befriedigende Lösung aus. In Bezug auf die erste Lösungsmöglichkeit können nämlich Wandstärken von mehr als 200 µm aufgrund der Gewährleistung einer leichten Einführbarkeit der Stents und der begrenzten Gefäßabmessungen nicht vertreten werden. Für den zweiten Fall ist nur ein sehr beschränktes Spektrum aus biokompatiblen und mäßig schnell degradierbaren Legierungen bekannt. Im Hinblick auf die dritte Lösungsmöglichkeit sind lediglich Passivierungen aus Fluor bekannt.

Die zuvor genannten Passivierungsschichten weisen zwei grundsätzliche Nachteile auf, die sich u. a. dadurch ergeben, dass derartige Stents üblicherweise zwei Zustände annehmen, nämlich einen komprimierten Zustand mit einem kleinen Durchmesser und einen expandierten Zustand mit einem größeren Durchmesser. Im komprimierten Zustand kann der Stent mittels eines Katheters in das zu stützende Gefäß eingeführt und an der zu behandelnden Stelle positioniert werden. Am Ort der Behandlung wird der Stent dann beispielsweise mittels eines Ballonkatheters dilatiert bzw. (bei Verwendung einer Formgedächtnislegierung als Stentmaterial) beispielsweise durch Erwärmung über eine Sprungtemperatur in den expandierten Zustand überführt. Aufgrund dieser Durchmesseränderung ist das Grundgitter des Stents hierbei einer mechanischen Belastung ausgesetzt. Weitere mechanische Belastungen des Stents können während der Herstellung oder bei der Bewegung des Stents im oder mit dem Gefäß, in das der Stent eingesetzt ist, auftreten. Bei den genannten Passivierungen ergibt sich somit der Nachteil, dass während der Verformung des Implantats Mikrorisse entstehen, die zur Unterwanderung des Beschichtungsmaterials führen, so dass die Passivierungswirkung der Beschichtung herabgesetzt wird. Hierdurch wird eine unspezifizierte lokale Degradation verursacht. Außerdem sind das Einsetzen und die Geschwindigkeit der Degradation von der Größe und der Verteilung der Mikrorisse abhängig, die als Fehlstellen schlecht kontrollierbar sind. Dies führt zu einer starken Streuung in den Degradationszeiten.

In der Druckschrift WO 2005/065576 A1 wird die Degradationssteuerung degradierbarer Implantate durch eine Beschichtung aus einem biodegradierbaren Werkstoff offenbart. Eine ortsabhängige Degradation des Implantats wird dadurch optimiert, dass der Grundkörper eine in vivo ortsabhängige erste Degradationscharakteristik besitzt und eine den Grundkörper vollständig oder ggf. nur bereichsweise bedeckende Beschichtung aus zumindest einem biodegradierbarem Werkstoff aufweist, wobei die Beschichtung in vivo eine zweite Degradationscharakteristik besitzt. Die an einem Ort kumulierte Degradationscharakteristik ergibt sich somit aus der Summe der jeweils an dem genannten Ort bestehenden Degradationscharakteristika von Werkstoff und Beschichtung. Die ortsabhängige kumulierte Degradationscharakteristik wird dabei durch die Variation der zweiten Degradationscharakteristik so vorgegeben, dass die Degradation an dem genannten Ort in einem vorgegebenen Zeitintervall mit einem vorgebbaren Degradationsverlauf stattfindet.

Die in der Druckschrift WO 2005/065576 A1 beschriebene Degradationscharakteristik der biodegradierbaren Beschichtung wird ganz allgemein insbesondere durch Variation der morphologischen Struktur der Beschichtung, der stofflichen Modifikation des Werkstoffs und/oder die Anpassung der Schichtdicke der Beschichtung erreicht. Hierbei wird unter morphologischer Struktur die Konformation und Aggregation der die Beschichtung bildenden Verbindungen verstanden. Als Beispiel für eine Beschichtung ist in der Druckschrift Hyaluronsäure angegeben.

In der US 2006/0224237 A1 wird ebenfalls ein Transplantat oder ein Stent mit einer Schutzschicht beschrieben, das dazu verwendet wird, Oberflächenstrukturen des Stents vor ihrer Zerstörung zu bewahren. Hierbei können die Oberflächenstrukturen aus einem oder mehreren Materialien gebildet werden, die in verschiedenen Umweltbedingungen mindestens teilweise aufgelöst, degradiert oder absorbiert werden.

Die in diesen Druckschriften genannten Möglichkeiten der Beeinflussung der Degradation beinhalten noch keine befriedigenden Lösungen im Hinblick auf Endoprothesen, die in dem genannten Zielkorridor degradieren. In der Druckschrift WO 2005/065576 A1 werden nur sehr allgemeine Prinzipien dargestellt, die keine konkreten Lösungsvorschläge im Hinblick insbesondere auf Magnesium-Stents liefern.

Die Druckschrift US 2007/0050009 A1 beschäftigt sich mit einem Stent, der eine Stützstruktur aus biodegradierbarem Material aufweist. Diese Stützstruktur ist zumindest teilweise mit einer Absorptionsvermeidungsschicht (absorption inhibitor layer) versehen, welche die Rate der Absorption der Stützstruktur verringert. Die Absorptionsvermeidungsschicht selbst wird ebenfalls durch die umgebenden Körperflüssigkeiten absorbiert. Auch mittels dieser bekannten Lösung ist lediglich eine sehr eingeschränkte und für viele Anwendungsfälle ungenügende Steuerung der Degradation des Stents möglich. Als Materialien für eine Absorptionsvermeidungsschicht werden beispielsweise Hyaluronsäure, Collagen oder Polyglycolsäure angegeben.

WO 2008/034474 offenbart einen Stent mit einer Amino-funktionalisierten Parylenschicht, einer Oligonukleotidschicht und/oder einer Oligopeptidschicht, die eine spezifische Bindungsaffinität für CD 34-posititve Zellen aufweist.

EP 2 018 882 offenbart eine Endoprothese mit einem Grundgitter bestehend aus einem zumindest weitestgehend biodegradierbaren Material und einer auf dem biodegradierbaren Material angeordneten Beschichtung. Das Grundgitter ist vollständig durch eine Beschichtung bis auf mindestens einen Degradationsbereich bedeckt, wobei der mindestens eine Degradationsbereich als Aussparung in der Beschichtung ausgebildet ist.

Als Schutzschicht, insbesondere zur Verhinderung von Restenosen oder Entzündungen nach der Implantation bzw. als Vorbehandlungsschicht zu einem Träger von bioaktiven Wirkstoffen werden in den Druckschriften DE 10 2005 039 126 A1 und US 2005/0196424 A1 u. a. Beschichtungen mit Parylene beschrieben. Eine Anwendung dieser Beschichtungen für die Degradationssteuerung wird nicht offenbart.

Die Aufgabe der vorliegenden Erfindung besteht somit darin, eine Endoprothese anzugeben, deren mechanische Stützwirkung einerseits über einen längeren Zeitraum bestehen bleibt und deren Degradation andererseits zu einem kontrollierbaren Zeitpunkt, insbesondere in dem genannten Zielkorridor stattfindet. Zudem soll die Degradation den geometrischen Gegebenheiten des Stentdesigns und den damit verbundenen klinischen Anforderungen angepasst werden können.

Die Aufgabe wird durch eine Endoprothese gelöst, deren Beschichtung Parylene, vorzugsweise mindestens überwiegend Parylene, besonders bevorzugt Parylene C oder Parylene N, enthält, wobei das Grundgitter im Wesentlichen vollständig, vorzugsweise vollständig, durch die Beschichtung bedeckt ist und wobei die Dicke der Beschichtung zwischen etwa 0,1 µm und etwa 10 µm, bevorzugt zwischen etwa 0,4 µm und etwa 7 µm, besonders bevorzugt zwischen etwa 1 µm und etwa 5 µm beträgt, und die Parylenebeschichtung Bereiche aufweist, in der die Schichtdicke lokal gegenüber den übrigen Bereichen verringert ist.

Bei einer Schichtdicke der Parylene enthaltenden Schicht von mehr als 10 µm führt die Beschichtung aufgrund der großen Dicke zu einer nennenswerten Verringerung des blutdurchflossenen Lumens im Gefäß des Behandelten (u. a. auch durch die induzierte Neointimabildung). Bei einer Schichtdicke von weniger als 0,1 µm bilden sich in der Beschichtung Inhomogenitäten bezogen auf die Dicke der Schicht sowie Fehlstellen. Hierdurch kann die Degradation des darunter liegenden Endoprothesen-Grundgitters nicht mehr zuverlässig verhindert werden bzw. die Degradation verläuft mit einer zu großen und unerwünschten Variabilität.

Die Beschichtung mit Parylene, vorzugsweise die Beschichtung, welche zu mindestens 30 Gew.% aus Parylene, besonders bevorzugt zu mindestens 60 Gew.% aus Parylene, höchstbevorzugt zu mindestens 90 Gew.% aus Parylene, jeweils bevorzugt aus Parylene C und/oder Parylene N, besteht, beeinflusst das Degradationsverhalten positiv. Dies wird insbesondere dadurch erreicht, dass Parylene in überraschender Weise eine Diffusionsbremse darstellt, die - abhängig von der Schichtdicke der Beschichtung mit Parylene - die Diffusion von Wasser und/oder ggf. anderen Ionen oder Molekülen der umgebenden Körperflüssigkeit, z.B. Chloridionen, welche bei metallischen Grundgittern eine stark beschleunigende Wirkung auf die Degradation durch einen Angriff der Oxid- bzw. Passivierungsschicht haben, zur Oberfläche des Grundgitters gegenüber einem unbeschichteten Grundgitter einschränkt. Dabei kann im Fall von magnesiumhaltigen Grundgittern der durch die Degradation von Magnesium entstehende Wasserstoff aufgrund seiner geringen Molekülausdehnung und nicht polaren Bindung ohne nennenswerte Blasenbildung von der Oberfläche des Grundgitters durch die Beschichtung nach außen diffundieren. Außerdem wird die Degradation des Grundgitters dadurch abgebremst und/oder homogenisiert, dass die bei dem lokalen Abbau eine Rolle spielende Zellen (insbesondere Makrophagen) nicht direkt die Oberfläche des Grundgitters erreichen können. Hierdurch kann die Geschwindigkeit der Degradation entsprechend den klinischen Anforderungen gesteuert werden.

Die erfindungsgemäße Parylene enthaltende Beschichtung ist außerdem in vorteilhafter Weise flexibel ausgebildet. Hierbei bedeutet das Merkmal, dass die Beschichtung flexibel ausgebildet ist, dass die Beschichtung die Bewegung des Grundgitters nachvollzieht, so dass sich im Wesentlichen keine größeren Risse oder dergleichen in dem Beschichtungsmaterial ausbilden. Das bedeutet, dass das Material der Beschichtung selbst keine Stützfunktion aufweist, d. h. dass die Beschichtung elastisch und somit biegbar ausgebildet ist. Die Elastizität und Flexibilität von Körper und Beschichtung ist umso größer, je stärker das Grundgitter, das unter der Beschichtung angeordnet ist, bereits degradiert ist, wobei die Degradation sich (in verringerter Geschwindigkeit) unterhalb der Beschichtung fortsetzt. Nach vollständiger Degradation bewegt sich die flexible Beschichtung, die selbst keine Stützfunktion hat, flexibel mit dem behandelnden Gefäß, in dessen Wand sie typischerweise durch Endothelialisierung und teilweise auch Neointimaproliferation eingebettet ist, und der darin strömenden Körperflüssigkeit.

Die Parylene enthaltende erfindungsgemäße Beschichtung ist demnach flexibel und besitzt außerdem ein geringes Quellvolumen. Somit kann der gewünschte Integritätsverlust auf die gewünschte Zeit, insbesondere vier Wochen bis sechs Monate, eingestellt werden.

Als Parylene werden vollständig lineare, teilkristalline und unvernetzte aromatische Polymere bezeichnet. Diese Polymere lassen sich, je nach ihrem Aufbau, in vier verschiedene Grundtypen einteilen, nämlich Parylene C, Parylene D, Parylene N und Parylene F.

Durch die Parylene enthaltende Beschichtung wird die Oberfläche der Endoprothese, d. h. des Grundgitters, von der Deckschicht derart geschützt, dass sie im Gegensatz zu bekannten Passivierungen die mechanischen Beanspruchungen wie Crimpen, Dilatieren oder Crossing der Läsion übersteht, ohne dass sich ungewollt Risse oder andere Fehlstellen ausbilden. Hierdurch wird eine unkontrollierte Degradation der Endoprothese an Stellen, an denen diese nicht gewünscht ist, verhindert. Durch die Vermeidung einer ungesteuerten Riss- oder Fehlstellenbildung bei einer flexiblen Beschichtung kann eine starke Streuung der Degradationszeiten verhindert werden.

Bei der Degradation einer erfindungsgemäßen Endoprothese diffundiert das degradierte Endoprothesenmaterial zumindest teilweise durch die Beschichtung mit Parylene hindurch und es bleibt ein dünner Schlauch aus der Beschichtung ggf. Abbauprodukte des degradierten Endoprothesenmaterials enthaltend bestehen. Bei einer Endoprothese aus einer Magnesium-Legierung entstehen beispielsweise weiche Magnesiumabbauprodukte bzw. - umbauprodukte wie Calciumphosphat (aus dem körpereigenen Puffersystem), sowie ggf. Magnesiumhydroxid oder auch Magnesiumphosphat. Das Auftreten von solchen Produkten ist bei der Auswahl geeigneter biokompatibler Legierungen klinisch akzeptabel.

Die Beschichtung mit Parylene ist bevorzugt mittels eines Plasmabeschichtungsverfahrens aufbringbar, wobei sich das Material durch eine hohe Spaltgängigkeit auszeichnet, so dass das Grundgitter vollständig bedeckt werden kann. Die Dicke der vorzugsweise mittels des Plasmabeschichtungsverfahrens aufgebrachte Beschichtung mit Parylene, vorzugsweise der Beschichtung mit Parylene C oder Parylene N, beträgt zwischen etwa 0,1 µm und etwa 10 µm, bevorzugt zwischen etwa 0,4 µm und etwa 7 µm, besonders bevorzugt zwischen etwa 1 µm und etwa 5 µm. Bei einer Schichtdicke oberhalb von 10 µm wird die Beschichtungszeit zu lang, so dass das Beschichtungsverfahren zu kostenaufwendig wird.

Die Parylenebeschichtung weist Bereiche auf, in der die Schichtdicke lokal gegenüber den übrigen Bereichen verringert ist. Hierbei liegt die Schichtdicke in den Bereichen mit verringerter Schichtdicke jedoch noch in dem oben angegebenen Schichtdicken-Wertebereich, wobei die Bereiche geringerer Schichtdicke beispielsweise eine Dicke von größer als 0% bis etwa 70%, besonders bevorzugt von etwa 5% bis etwa 50% der Dicke in den übrigen Bereichen aufweisen.

Durch die Bereiche mit verringerter Schichtdicke, die an verschiedenen Stellen des Grundgitters (Beispiele siehe unten) angeordnet sein können, ist es möglich, die räumliche Verteilung des Beginns eines etwas beschleunigten Degradationsangriffs zu steuern, da in den Bereichen mit geringerer Schichtdicke die Degradation schneller abläuft. Zum Beispiel ist es in vielen Fällen geeignet, wenn die Längsverbinder (Verbindungsstege) des Stents deutlich schneller degradieren als die stützenden, ringförmigen bzw. spiralförmigen Elemente. Zur Herstellung der Bereiche mit verringerter Schichtdicke kann die Beschichtung mittels der unten beschriebenen Verfahren strukturiert werden. Hierdurch entstehen beispielsweise Mulden oder Riefen, die lateral eine makroskopische oder mikroskopische Ausdehnung (typischerweise zwischen 1 µm und 1 mm) aufweisen. Die geometrische Strukturierung wird so gewählt, dass sich die gewünschte Degradationskinetik der Endoprothese (lokal kontrolliert) ergibt.

Die Parylene-Beschichtung kann auch in so dünnen Schichten (je nach Material und Beschaffenheit der Grundgitteroberfläche mit einer Dicke von etwa 0,1 µm bis 1 µm) aufgebracht werden, dass die Schicht nicht vollständig geschlossen ist, sondern sich noch in einer Phase des Inselwachstums befindet, so dass die zwischen den Inseln aus der Parylene enthaltenden Beschichtung Aussparungen oder dünnere Bereiche der Schicht durch die nicht bzw. kaum beschichteten Oberflächenanteilen gebildet werden. In diesen Bereichen findet der Degradationsangriff nach der Implantation der erfindungsgemäßen Endoprothese zuerst statt.

Alternativ können bestimmte, vordefinierte Bereiche geringerer Schichtdicke von der Beschichtung mit Parylene auch dadurch erzeugt werden, dass sich die bestimmten Bereiche durch eine spezielle Ausgestaltung des Stentkörpers, beispielsweise durch einen geringeren Durchmesser, beim Dilatieren des Stents in der Läsion an der Oberfläche deutlich mehr verformen als andere Bereiche. Hierdurch wird die Paryleneschicht an diesen Stellen in die Länge gezogen und somit ausgedünnt.

Auf die Parylene enthaltende Beschichtung kann in einem Ausführungsbeispiel der vorliegenden Erfindung eine weitere Schicht aufgebracht sein, welche einen Carrier (Träger) und mindestens eine pharmazeutisch aktive Substanz enthält. Der Träger nimmt dabei gewissermaßen die pharmazeutisch aktive Substanz auf.

Unter einer "pharmazeutisch aktiven Substanz" (oder therapeutisch aktive oder wirksame Substanz, Medikament, Wirkstoff) im Sinne der Erfindung wird ein pflanzlicher, tierischer oder synthetischer Wirkstoff (Medikament) oder ein Hormon verstanden, das in geeigneter Dosierung als Therapeutika zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiphlogistika (wie beispielsweise Cortison oder Dichlofenac), der Antiinflammatorica (wie beispielsweise Imidazole), der Antiallergika, der Oligonucleotide (wie beispielsweise dODN), der Estrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, Analgetika, Antirheumatika und Zytostatika, vorzugsweise Cyclosporin A, der Taxole oder Taxane, hier vorzugsweise Paclitaxel oder Limus-Verbindungen, vorzugsweise Sirolimus (Rapamycin), Zotarolimus, Tacrolimus, Biolimus, Everolimus bestehen.

Die Dicke der mindestens einen Wirkstoff tragenden Schicht beträgt etwa 0,1 µm bis etwa 40 µm, bevorzugt zwischen etwa 0,6 µm und etwa 15 µm, besonders bevorzugt zwischen etwa 1 µm und etwa 10 µm. Die Schicht kann aus mehreren Einzelschichten aufgebaut sein. Die Beladung mit Medikament beträgt etwa 1 Gew.% bis 90 Gew.% bevorzugt zwischen etwa 5 Gew.% und etwa 80 Gew.%, besonders bevorzugt zwischen etwa 10 Gew.% und etwa 60 Gew.%.

Der Vorteil dieses Ausführungsbeispiels besteht darin, dass durch die Parylene enthaltende Beschichtung die medikamentenbeladene Schicht effektiv von der Degradation des Stents getrennt wird, so dass das Medikament wirksam im Gefäß eluiert werden kann. Um die Wirkung des Stents noch zu verbessern, indem Proliferation und/oder Inflammation eingeschränkt werden, lässt sich ein geeignetes Medikament (z. B. Paclitaxel oder Sirolimus und ihre Derivate) auf den Stent aufbringen, dass dann über einen geeigneten Zeitraum in vivo eluiert wird. Dies kann durch die Einbringung in Oberflächenkavitäten oder eine Trägersubstanz (typischerweise ein Polymer) erreicht werden. Bei der Nutzung solcher Medikamente auf degradierbaren Stents ergibt sich der zusätzliche Vorteil, dass die chemische Wirkung von Abbauprodukten des Grundkörpers (z. B. OH--Ionen bei Mg oder H+-Ionen bei PLLA) auf das Medikament und die Medikamentenelution in geeigneter Weise so verringert wird, dass eine ausreichende, effektive Menge des Medikaments eluiert wird und das Medikament nicht zu stark durch die degradationsbedingte pH-Wert-Verschiebung oder andere bei der Degradation ablaufende chemische Prozesse zerstört bzw. nennenswert in seiner Wirksamkeit beeinträchtigt wird.

Vorteilhafte Polymercarrier sind beispielsweise Poly-D,L-lactid, PEVA-PBMA (Cypher), SIBS (Taxus) oder Polyethersulfon. Auch Fette können als Carrier (Träger, Matrix) eingesetzt werden. Weitere Polymere, die in der wirkstoffhaltigen Schicht enthalten sind, werden vorzugsweise ausgewählt aus der Gruppe bestehend aus:
- nicht degradierbaren Polymeren, beispielsweise Polyethylen; Polyvinylchlorid; Polyacrylate; vorzugsweise Polyetyl- und Polymethylacrylate, Polymethylmetacrylat, Polymethyl-co-ethylacrylat und Ethylen/Ethylacrylat; Polytetrafluorethylen, vorzugsweise Ethylen/Chlortrifluorethylen Copolymere, Ethylen/Tretrafluorethylen Copolymere; Polyamide, vorzugsweise Polyamidimid, PA-11, PA-12, PA-46, PA-66; Polyetherimid; Polyethersulfon; Poly(iso)butylen; Polyvinylchlorid; Polyvinylfluorid; Polyvinylalkohol; Polyurethan; Polybuthylenterephthalat; Silikone; Polyphosphazen; Polymerschäume, vorzugsweise Polymerschäume aus Carbonaten, Styrolen; Copolymere und/oder Blends der aufgezählten Polymerklassen, Polymere der Klasse der Thermoplaste,
- degradierbaren Polymeren, beispielsweise Polydioxanon; Polyglycolid; Polycaprolacton; Polylactide, vorzugsweise Poly-L-Lactid, und Copolymere sowie Blends hiervon, vorzugsweise Poly(L-Lactid-co-glycolid), Poly(D,L-lactid-co-glycolid), Poly(L-Lactid-co-D,L-Lactid), Poly(1-Lactid-co-trimethylen carbonat); Triblockcopolymere; Polysaccharide, vorzugsweise Chitosan, Levan, Hyaluronsäure, Heparin, Dextran, Cellulose; Polyhydroxyvalerat; Ethylvinylacetat; Polyethylenoxid; Polyphosphorylcholin; Fibrin; Albumin; Polyhydroxybuttersäure, vorzugsweise ataktische, isotaktische und/oder syndiotaktische Polyhydroxybuttersäure sowie deren Blends.

Die optionale Medikamente tragende Schicht wird auf die Parylene enthaltende Schicht vorzugsweise durch einen einfachen Sprühprozess aus einer Lösung aufgebracht. Aber auch andere Aufbringungsverfahren wie Dippen, Pipettieren o. ä. können angewendet werden.

Falls die Parylene enthaltende Schicht Bereiche aufweist, die eine verringerte Dicke haben, dann wird die einen Wirkstoff tragende Schicht vorzugsweise derart vorgesehen, dass sich die den Wirkstoff tragende Schicht nicht dort angeordnet ist, wo die Bereiche mit der verringerten Schichtdicke liegen.

Wie bereits oben angegeben wurde, ist insbesondere eine Parylene enthaltende Beschichtung vorzugsweise mittels eines Plasmabeschichtungsverfahrens aufbringbar. Es können sowohl Gas- als auch elektrolytische Plasmaprozesse verwendet werden. Hierbei kann das Plasma mit geeigneten technischen Einrichtungen (Abschirmung, Gasstrom, Gegenelektrodenform etc.) gezielt zur Bearbeitung einzelner Endoprothesen bzw. ihrer Geometriesegmente eingestellt werden.

Zur Modifikation der Beschichtung, z. B. zur Variation der Schichtdicke über die Endoprothesen-Oberfläche oder die Rauhigkeit der Oberfläche, können beispielsweise Laser-, Elektronen- oder Ionenstrahlung oder elektromagnetische Felder eingesetzt werden. Lokal können die Degradationsbereiche auch durch eine Bestrahlung mit flüchtigen Festkörpern (z. B. Trockeneis) behandelt werden, so dass die Beschichtung an den behandelten Stellen lokal in einem Oberflächenbereich versprödet. Anschließend kann mittels anderer Prozesse, beispielsweise mittels Laser-, Elektronen- oder Ionenbestrahlung ein oberflächlicher Bereich der Schicht entfernt werden, so dass ein Bereich mit geringerer Schichtdicke verglichen mit den übrigen Bereichen entsteht. Modifizierte Bereiche können auch durch eine Bestrahlung mit Festkörpern (Sand, Keramik, Magnesium, Salze usw.) oder Flüssigkeiten (Wasserstrahl, Öle, Säure, Fette) oder Festkörper-/Flüssigkeitsgemischen erzeugt werden. Ebenso können Degradationsbereiche durch mechanische Bearbeitung der Schicht (beispielsweise mittels Nadeln, Bürstensystemen) in Trommeln beziehungsweise durch Gleitschleifverfahren (Trowalisieren) hergestellt werden.

Bei den vorgenannten Herstellungsverfahren können entsprechende Optiken, welche an die entsprechende Endoprothesengeometrie angepasst sind, verwendet werden. Im Fall der Laserbestrahlung können Faseroptiken verwendet werden. Weiter können hochdynamische Handhabungstechniken eingesetzt werden und mittels Masken Bereiche der beschichteten Endoprothesenoberfläche, welche nicht bearbeitet werden soll, abgeschirmt werden.

Bei einem besonders bevorzugten Herstellungsverfahren einer Parylene enthaltenden Beschichtung mit Bereichen geringerer Schichtdicke werden diese durch ein sich an das Aufbringen der Beschichtung anschließende Ätzen in einem Sauerstoffplasma erzeugt. Beschichtungen mit Parylene des Typs C und N führen in der Regel zu einer makroskopisch gleichmäßigen Bedeckung der Oberfläche der Endoprothese. Mikroskopisch existieren bei beiden Schichtvarianten jedoch verteilt über die Oberfläche der Endoprothese Schichtdickenunterschiede im Bereich einiger 0,1 µm.

Wird eine mit einer Parylene enthaltenden Beschichtung von bevorzugt 1 bis 5 µm Dicke bedeckte Oberfläche einer Endoprothese einer Sauerstoffplasmabehandlung unterzogen, so wird die Beschichtung durch die Sauerstoff-Ionen attackiert. Dies hat eine örtlich selektive Verringerung der Parylene enthaltenden Beschichtung zur Folge. Dieser Verlust an schützender Wirkung der Beschichtung ist umgekehrt proportional zur Schichtdicke. Die Verfahrensparameter (z. B. Sauerstoffpartialdruck, Einwirkzeit, Kammertemperatur) des Sauerstoffplasmas werden dabei entsprechend gesteuert, dass die Schwachstellen der Beschichtung an ausgewählten Stellen entstehen. Hierdurch wird eine stellenweise beschleunigte Degradation des Endoprothesenmaterials erreicht, so dass der Ort der Degradation gesteuert werden kann. Zum Ätzen der Beschichtung können auf analoge Weise auch die Verfahren Plasmaätzen, Reactive-Ion-Etching und Deep-Reactive-Ion-Etching angewendet werden.

Als weitere Möglichkeit kann auf die Beschichtung auch ein Resist aufgebracht werden. Dieser Resist wird so strukturiert, dass gezielt nur die Beschichtung an bestimmten Stellen (Sollbruchstellen) abgetragen und damit in ihrer Schichtdicke verringert wird. Der Resist wird nachträglich nasschemisch wieder abgelöst.

Als weitere Herstellungs-Variante ist eine spezielle Formgebung/Bearbeitung von degradierbaren Endoprothesen möglich, die Schwachstellen in der später aufgebrachten Parylene-Schicht erzeugt. In dieser Variante entstehen Sollbruchstellen noch nicht bei der eigentlichen Produktion der Endoprothese sondern erst im Laufe der Implantation dieser. Diese Schwachstellen (Sollbruchstellen) werden z.B. durch Löcher bzw. Makroporen in den Stegen eines Stents realisiert, die vorzugsweise mittels Laserschneiden eingebracht werden. Dieser Verfahrensschritt wird im Verlauf des üblichen Laserschneidprozesses zur Anwendung gebracht. Die nachfolgende Parylene-Beschichtung führt zwar zunächst zu einem auch diese Sollbruchstellen versiegelnden Effekt. Allerdings kommt es bereits beim Dilatieren des Stents zur Mikrorissbildung bevorzugt in der Nähe der Zonen mit den Sollbruchstellen. Diese Mikrorisse treten vor allem in den sich durch höchste Spannungskonzentrationen auszeichnenden Gebieten um die Sollbruchstellen auf. Dort findet dann der bevorzugte Korrosionsangriff statt. Dieser hat insbesondere die Eigenschaft, dass er zeitlich gleichmäßig und gleich stark in den Zonen um die Sollbruchstelle stattfindet. Das Korrosionsmedium dringt in die mikrorissbehaftete Parylene-Schicht ein, korrodiert das darunter angeordnete degradierbare Material des Grundgitters und führt letztendlich zu einer wochengenau kalkulierbaren Querschnittsschwächung der Stentstege durch Korrosionsangriff. Alternativ entstehen in der Parylene-Schicht Sollbruchstellen an den Teilen des Stents, die sich schon konstruktiv bedingt an der Oberfläche durch Crimpen und Aufdilatieren stark verformen.

In einem weiteren Ausführungsbeispiel der Erfindung ist eine Haftvermittlerschicht zwischen der inerten Beschichtung und dem Material des Grundgitters vorgesehen. Eine derartige Haftvermittlerschicht verbessert die Haftung zwischen der Beschichtung und dem Material des Grundgitters. Eine derartige Haftvermittlerschicht kann beispielsweise eine oder mehrere der Verbindungen aus der Gruppe der anorganischen Magnesium-Verbindungen (Magnesiumoxid, Magnesiumphosphat, etc.) oder der Calciumphosphate aufweisen.

Bei einem Aufbau der Endoprothese aus Stützelementen, die vorzugsweise zick-zack-, mäander- oder spiralförmig ausgebildet sind und die Abstützung des Gefäßes oder anderer Hohlorgane übernehmen, und aus diese Stützelemente verbindenden Verbindungsstegen, die selbst keine stützende Funktion übernehmen, ist in einem weiteren besonders bevorzugten Ausführungsbeispiel eine Vielzahl von Bereichen mit geringerer Schichtdicke lediglich im Bereich der Verbindungsstege angeordnet. Beispielsweise wird je ein Bereich geringerer Schichtdicke lediglich in der Mitte eines Verbindungsstegs angeordnet. Eine derartige Ausführungsform ist besonders einfach aufgebaut und kann außerdem mit geringen Produktionskosten realisiert werden. Eine derartige Endoprothese hat den Vorteil, dass ihr Kollapsdruck nach einem gewünschten Zeitraum wie 4 Wochen bis 6 Monate sehr schnell abfällt. Eine solche Endoprothese ist für den klinischen Einsatz besonders gewünscht.

Die Erfindung wird nachfolgend anhand von mittels Figuren dargestellten Ausführungsbeispielen näher erläutert. Dabei bilden alle beschriebenen und/oder bildlich dargestellten Merkmale den Gegenstand der Erfindung, unabhängig von ihrer Zusammenfassung in den Ansprüchen oder deren Rückbeziehung.

Es zeigen schematisch:
- Figur 1: einen Abschnitt eines ersten Ausführungsbeispiels einer erfindungsgemäßen Endoprothese in einem Querschnitt,
- Figur 2: einen Abschnitt eines zweiten Ausführungsbeispiels einer erfindungsgemäßen Endoprothese in einem Querschnitt,
- Figur 3: einen Abschnitt eines dritten Ausführungsbeispiels einer erfindungsgemäßen Endoprothese in einem Querschnitt,
- Figur 4: einen Abschnitt eines vierten Ausführungsbeispiels einer erfindungsgemäßen Endoprothese in einer Ansicht von der Seite,
- Figur 5: die Strukturformeln von Parylene C (Figur 5a) und Parylene N (Figur 5b),
- Figur 6: einen Abschnitt eines fünften Ausführungsbeispiels einer erfindungsgemäßen Endoprothese in einer Ansicht von der Seite,
- Figur 7: einen Abschnitt eines sechsten Ausführungsbeispiels einer erfindungsgemäßen Endoprothese in einem Querschnitt, mit einer zusätzlichen Schicht, die einen Wirkstoff aufweist, sowie
- Figuren 8 und 9: einen Querschnitt eines Abschnitts der Stützstruktur eines siebten Ausführungsbeispiels einer erfindungsgemäßen Endoprothese.

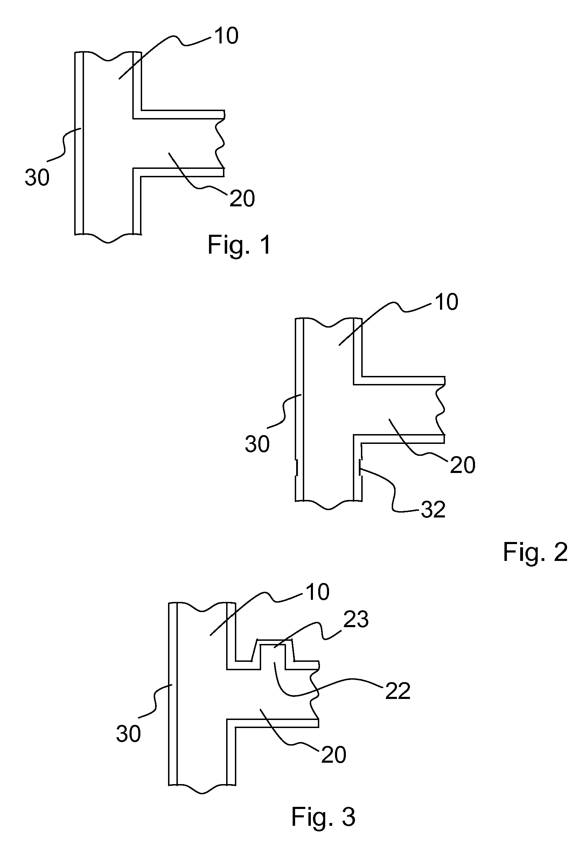

Figur 1 zeigt einen Abschnitt eines Grundgitters einer erfindungsgemäßen Endoprothese, die als Stent ausgebildet ist. Das Grundgitter weist zick-zack- oder mäanderförmig gefaltete, im Wesentlichen in Umfangsrichtung verlaufende oder helixförmige Stege als Stützelemente 10 sowie im Wesentlichen in Längsrichtung des Stents verlaufende Stege als Verbindungsstege 20 auf. Der Stent ist insgesamt als eine in Richtung der Verbindungsstege 20 verlaufende rohrförmige oder hohlzylinderförmige Endoprothese, die an ihren Enden offen ist, ausgebildet. In Figur 1 ist lediglich ein Abschnitt des Grundgitters dargestellt, in dem das Ende eines Verbindungsstegs 20 an ein Stützelement 10 stößt.

Das Grundgitter des Stents besteht zumindest überwiegend aus einem oder mehreren, oben angegebenen weitestgehend biodegradierbaren Materialien, vorzugsweise aus Magnesium oder einer Magnesiumlegierung, besonders bevorzugt WE43. Das Grundgitter weist auf seiner gesamten Oberfläche eine das Grundgitter vollständig bedeckende Parylene enthaltende Beschichtung 30 mit einer im Wesentlichen konstanten Schichtdicke auf. Die Schichtdicke beträgt zwischen etwa 0,1 µm und etwa 10 µm, bevorzugt zwischen etwa 0,4 µm und etwa 7 µm, besonders bevorzugt zwischen etwa 1 µm und etwa 5 µm. Als Materialien für die Beschichtung 30 kommen beispielsweise Parylene C oder Parylene N in Frage, wobei die Beschichtung 30 vorzugsweise vollständig oder zumindest zu 90 Gew.% aus Parylene C oder Parylene N besteht.

Bei dem in Figur 2 dargestellten zweiten Ausführungsbeispiel eines erfindungsgemäßen Stents ist im Bereich des Stützelements 10 ein ringförmiger, um das Stützelement 10 herum laufender Bereich 32 vorgesehen, in dem die Beschichtung 30 eine verringerte Schichtdicke aufweist. Durch einen derartigen Bereich 32 kann genau gesteuert werden, wo die Degradation des erfindungsgemäßen Stents mit einer höheren Geschwindigkeit verläuft.

Das in Figur 3 dargestellte Ausführungsbeispiel weist an einer Vielzahl von Verbindungsstegen 20 einen Bereich 22 in der Form eines fingerförmigen Fortsatzes auf, der aus dem Material des Stents besteht. Der fingerförmige Fortsatz 22 hat eine im Wesentlichen zylindrische Form, die sich in einem weiteren, nicht dargestellten Ausführungsbeispiel in die von dem Grundgitter weg weisende Richtung verjüngen, d. h. ihren Durchmesser verringern, kann. Die Beschichtung 30 ist an dem von dem Verbindungssteg 20 wegragenden Ende 23 mit einer geringeren Schichtdicke ausgeführt. Wie bei dem zweiten Ausführungsbeispiel wird hierdurch im Bereich des Endes 23 des Fortsatzes 22 eine schnellere Degradation des Materials der Endoprothese bewirkt.

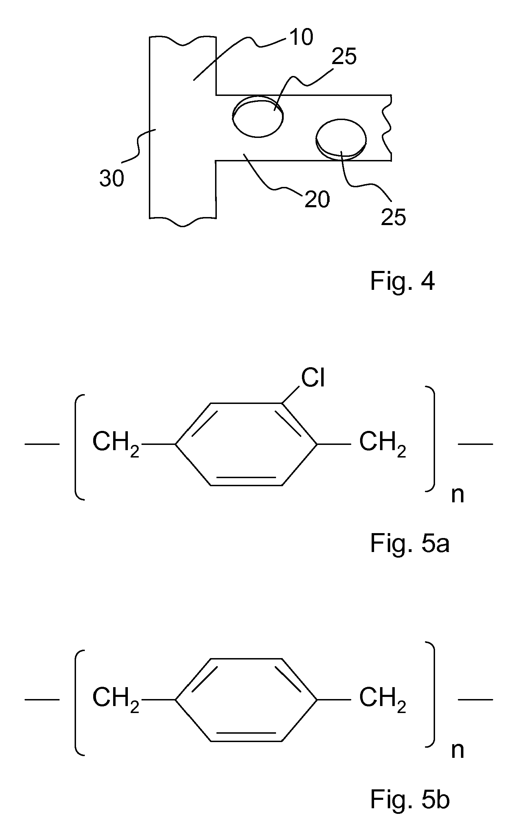

In dem anhand von Figur 4 dargestellten vierten Ausführungsbeispiel sind vorzugsweise an den Verbindungsstegen 20 angeordnete kreisrunde Bereiche 25 vorgesehen, in denen die Beschichtung 30 eine geringere Schichtdicke aufweist. Auch an diesen Stellen wird die Degradation des Stents nach dem Einsetzen in den Körper schneller erfolgen.

Die gezeigten Ausführungsbeispiele für die Anordnung der Bereiche mit einer geringeren Schichtdicke können beliebig entsprechend des gewünschten Degradationsverhaltens variiert werden. Demzufolge können die fingerförmigen Fortsätze 22 auch an den Stützelementen 10 oder an anderen Stellen der Verbindungsstege 20 angeordnet sein. Zudem können die fingerförmigen Fortsätze 22 auch mehrfach an den Stützelementen oder lediglich an bestimmten Stützelementen 10 bzw. Verbindungsstegen 20 angeordnet sein. Gleiches gilt auch für die ringförmige Bereiche 32 oder den kreisrunden Bereich 25. Die verschiedenen Formentypen der Bereiche mit geringerer Schichtdicke können beliebig variiert und/oder beliebig miteinander an einer Endoprothese kombiniert werden.

Die Endoprothesen können dadurch hergestellt werden, dass zunächst die Endoprothese mit den bekannten Herstellungsverfahren aus dem biodegradierbaren Material hergestellt wird. Gegebenenfalls sind hierbei an dem Grundgitter an den gewünschten Stellen die fingerförmigen Fortsätze 22 oder andere Degradationselemente vorzusehen. Anschließend wird die Beschichtung 30 mittels bekannter Beschichtungsverfahren (zum Beispiel für Parylene mittels eines Plasmabeschichtungsverfahrens bzw. Gasphasenabscheidungsverfahren) aufgebracht, wobei an den Stellen, an denen Degradationsbereiche mit geringerer Schichtdicke vorgesehen werden sollen, während der Beschichtung teilweise eine Abdeckung angeordnet wird, so dass die Beschichtung während des Beschichtungsprozesses in diesen Bereichen nicht teilweise aufgetragen wird. Hierfür können beispielsweise Schablonen verwendet werden. Anschließend wird die Abdeckung entfernt. Alternativ kann die Beschichtung auch zunächst auf die gesamte Oberfläche der Endoprothese gleichmäßig bzw. homogen aufgebracht und anschließend in einigen Bereichen teilweise wieder entfernt werden, so dass die Oberfläche des Stents strukturiert ist.

Die Strukturformeln von Parlyene C und Parylene N, welche jeweils ein bevorzugtes Material der Beschichtung 30 darstellen, sind in Figur 5a bzw. in Figur 5b gezeigt.

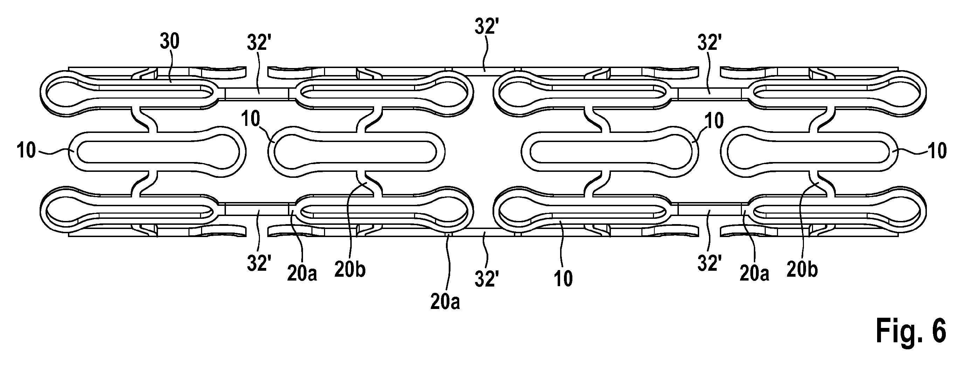

In Figur 6 ist noch einmal ein längerer Abschnitt einer erfindungsgemäßen Endoprothese in Form eines Stents dargestellt, welche an den in Längsrichtung verlaufenden Verbindungsstegen 20a der Stützelemente 10 ringförmige und umlaufende Bereiche 32' mit geringerer Schichtdicke der Beschichtung 30 aufweisen, die sich fast über die gesamte Länge dieser Verbindungsstege 20a erstrecken. Die nicht in Längsrichtung sondern geschwungen in im Wesentlichen radialer Richtung verlaufenden Verbindungsstege 20b der Stützelemente 10 weisen keine Bereiche geringerer Schichtdicke auf.

Die Bereiche geringerer Schichtdicke weisen eine Schichtdicke von 0% bis 70%, bevorzugt 5% bis 50%, der Schichtdicke in den übrigen Bereichen auf.

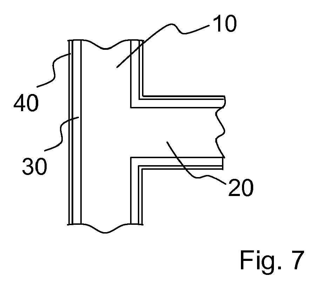

In Figur 7 ist ein weiteres Ausführungsbeispiel einer erfindungsgemäßen Endoprothese dargestellt, welche im Aufbau dem in Figur 1 dargestellten ersten Ausführungsbeispiel entspricht. Zusätzlich enthält die gezeigte Endoprothese eine weitere Schicht 40, welche einen Träger, vorzugsweise aus einem degradierbaren Polymer, sowie mindestens eine der oben genannten pharmazeutisch aktive Substanzen enthält und auf der Oberfläche der Parylene enthaltenden Beschichtung 30 angeordnet ist.

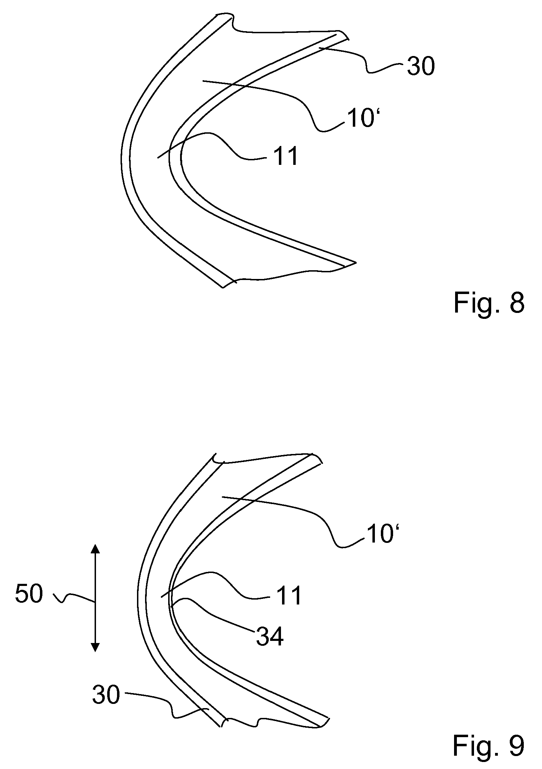

In Figur 8 wird ein Abschnitt eines Stützelements 10' eines siebten Ausführungsbeispiels der erfindungsgemäßen Endoprothese in Form eines Stents dargestellt. Der Ausschnitt zeigt eine Biegung oder Krümmung 11 eines Stützelements 10', wobei die Oberfläche des Stützelements 10' im Querschnitt einen Scheitel ausbildet. Im Bereich der Krümmung 11 weist das Stützelement 10' aus dem biodegradierbaren Material einen geringfügig kleineren Durchmesser auf als in den übrigen Bereichen. Auf der Oberfläche des Stützelements ist eine gleichmäßige, die Oberfläche vollständig bedeckende Parylene enthaltende Beschichtung 30 vorgesehen.

Bei der Dilatation der als Stent ausgeführten Endoprothese in der Läsion in Dilatationsrichtung 50 wird, wie in Figur 9 gezeigt, das Stützelement 10' aufgrund seiner dünneren Ausbildung des biodegradierbaren Materials im Bereich der Krümmung 11 stärker zurückgebogen als die übrigen Bereiche des Stützelements 10'. Durch die Dehnung in Richtung 50 (radiale Richtung des Stents) versucht das Stützelement 10' die Krümmung wieder zu begradigen. Durch die Zurückbiegung der Krümmung 11 wird auf der Innenseite, d.h. im konkaven Bereich der Krümmung 11, ein Bereich 34 der Beschichtung 30 stärker gedehnt, so dass nach Abschluss der Dilatation die Dicke der Beschichtung im Bereich 34 geringer ist als in den übrigen Bereichen. Hierdurch findet der Degradationsangriff nach der Implantation der erfindungsgemäßen Endoprothese im Bereich 34 zuerst statt.

In einem weiteren Ausführungsbeispiel kann im Bereich der Innenseite der Krümmung 11 zusätzlich oder alternativ zu dem in den Figuren 8 und 9 dargestellten Ausführungsbeispiel mit einem geringeren Durchmesser des Stützelements eine Kerbe angeordnet werden. Die Kerbe dient analog zur Ausgestaltung mit einem geringeren Durchmesser dazu, dass das Stützelement in diesem Bereich am weitesten zurückgebogen wird. Hierdurch wird ebenfalls erreicht, dass die darüber liegende, Parylene aufweisende Beschichtung in diesem Bereich am stärksten gedehnt wird, so dass eine stärkere Degradation in diesem Bereich erfolgt.

### Bezugszeichenliste:

- 10, 10': Stützelement
- 11: Krümmung des Stützelements
- 20, 20a, 20b: Verbindungssteg
- 22: fingerförmiger Fortsatz
- 23: wegragendes Ende des fingerförmigen Fortsatzes 22
- 25: kreisrunder Bereich mit geringerer Dicke der Beschichtung 30
- 30: Beschichtung
- 32, 32': ringförmiger Bereich mit geringerer Dicke der Beschichtung 30
- 34: Bereich mit geringerer Dicke der Beschichtung 30
- 40: einen Wirkstoff enthaltende Schicht
- 50: Dilatationsrichtung

## Patentansprüche

1. Endoprothese insbesondere intraluminale Endoprothese, z. B. ein Stent, mit einem Grundgitter bestehend aus einem zumindest weitestgehend biodegradierbaren Material und einer auf dem biodegradierbaren Material angeordneten Beschichtung (30), **dadurch gekennzeichnet, dass** die Beschichtung (30) Parylene, vorzugsweise mindestens überwiegend Parylene, besonders bevorzugt Parylene C oder Parylene N, enthält, und dass das Grundgitter im Wesentlichen vollständig durch die Beschichtung (30) bedeckt ist, wobei die Dicke der Beschichtung (30) zwischen 0,1 µm und 10 µm, bevorzugt zwischen 0,4 µm und 7 µm, besonders bevorzugt zwischen 1 µm und 5 µm beträgt, und wobei die Beschichtung Bereiche (25, 32, 32') aufweist, welche lokal eine verringerte Schichtdicke verglichen mit den übrigen Bereichen der Beschichtung haben.

2. Endoprothese nach Anspruch 1, **dadurch gekennzeichnet, dass** zwischen der Beschichtung (30) und dem Material des Grundgitters eine Haftvermittlerschicht angeordnet ist, die vorzugsweise eine oder mehrere Verbindungen der Gruppe der anorganischen Magnesiumverbindungen, z. B. Magnesiumoxid, Magnesiumphosphat, oder der Calciumphosphate enthält.

3. Endoprothese nach einem der vorhergehenden Ansprüche, **dadurch gekennzeichnet, dass** das biodegradierbare Material Mg oder eine Mg-Legierung, vorzugsweise WE43, oder biodegradierbare Polymere, vorzugsweise PLLA, enthält.

4. Endoprothese nach einem der vorgehenden Ansprüche, **dadurch gekennzeichnet, dass** die Beschichtung (30) zusätzlich eine oder mehrere Polymere der Gruppe bestehend aus Polyestern, vorzugsweise Polylactiden, und Polypeptiden aufweist.

5. Endoprothese nach einem der vorhergehenden Ansprüche, **dadurch gekennzeichnet, dass** auf der Beschichtung (30) zumindest teilweise eine Schicht (40) mit mindestens einer pharmazeutisch aktiven Substanz aufgebracht ist.

6. Endoprothese nach Anspruch 6, **dadurch gekennzeichnet, dass** die Schicht (40) mit der pharmazeutisch aktiven Substanz ein degradierbares Polymer als Carrier enthält, bevorzugt aus der Gruppe der Polyester.

7. Verfahren zur Herstellung einer Endoprothese nach einem der Ansprüche 1 bis 6, **gekennzeichnet durch** die folgenden Schritte:
- Bereitstellen des Grundgitters der Endoprothese
- Aufbringen einer Parylene enthaltenden Beschichtung auf die Oberfläche der Endoprothese derart, dass diese im Wesentlichen vollständig bedeckt ist, vorzugsweise mittels Gasphasenabscheidung, und
- Durchführung einer Sauerstoffplasmabehandlung.

8. Verfahren zur Herstellung einer Endoprothese nach einem der Ansprüche 1 bis 6, **gekennzeichnet durch** die folgenden Schritte:
- Bereitstellen des Grundgitters der Endoprothese,
- Einbringen von Sollbruchstellen in das Grundgitter und
- Aufbringen einer Parylene enthaltenden Beschichtung auf die Oberfläche der Endoprothese derart, dass diese im Wesentlichen vollständig bedeckt ist, vorzugsweise mittels Gasphasenabscheidung.

9. Verfahren nach Anspruch 7 oder 8, **dadurch gekennzeichnet, dass** eine 0,1 bis 10 µm, vorzugsweise 0,4 µm bis 7 µm, besonders bevorzugt 1 µm bis 5 µm dicke Parylene enthaltende Beschichtung aufgebracht wird.

10. Verfahren nach einem der Ansprüche 7 bis 9, **dadurch gekennzeichnet, dass** auf die Parylene enthaltende Beschichtung eine weitere Schicht, die mindestens eine pharmazeutisch aktive Substanz enthält, aufgetragen wird.

11. Verfahren nach Anspruch 10, **dadurch gekennzeichnet, dass** die weitere Schicht mit mindestens einer pharmazeutisch aktiven Substanz mittels Aufsprühen einer Lösung aufgebracht wird.

## Claims

1. An endoprosthesis, in particular an intraluminal endoprosthesis, for example a stent, having a basic mesh consisting of an at least predominantly biodegradable material, and a coating (30) arranged on the biodegradable material, **characterised in that** the coating (30) contains parylene, preferably at least predominantly parylene, particularly preferably parylene C or parylene N, and **in that** the basic mesh is substantially completely covered by the coating (30), wherein the thickness of the coating (30) is between 0.1 µm and 10 µm, preferably between 0.4 µm and 7 µm, particularly preferably between 1 µm and 5 µm, and wherein the coating has regions (25, 32, 32') in which the layer thickness is locally reduced compared to the other regions of the coating.

2. The endoprosthesis according to claim 1, **characterised in that** an adhesion-promoting layer is arranged between the coating (30) and the material of the basic mesh, said adhesion-promoting layer preferably containing one or more compounds from the group comprising inorganic magnesium compounds, for example magnesium oxide, magnesium phosphate, or calcium phosphates.

3. The endoprosthesis according to either one of the preceding claims, **characterised in that** the biodegradable material contains Mg or an Mg alloy, preferably WE43, or biodegradable polymers, preferably PLLA.

4. The endoprosthesis according to any one of the preceding claims, **characterised in that** the coating (30) additionally comprises one or more polymers from the group consisting of polyesters, preferably polylactides, and polypeptides.

5. The endoprosthesis according to any one of the preceding claims, **characterised in that** a layer (40) containing at least one pharmaceutically active substance is applied to the coating (30) at least in part.

6. The endoprosthesis according to claim 6, **characterised in that** the layer (40) containing the pharmaceutically active substance contains a degradable polymer as carrier, preferably from the group of polyesters.

7. A method for producing an endoprosthesis according to any one of claims 1 to 6, **characterised by** the following steps:
- providing the basic mesh of the endoprosthesis;
- applying a parylene-containing coating to the surface of the endoprosthesis in such a way that the endoprosthesis is substantially completely covered, preferably by means of gas-phase deposition, and
- carrying out an oxygen plasma treatment.

8. A method for producing an endoprosthesis according to any one of claims 1 to 6, **characterised by** the following steps:
- providing the basic mesh of the endoprosthesis,
- introducing predetermined breaking points into the basic mesh, and
- applying a parylene-containing coating to the surface of the endoprosthesis in such a way that the endoprosthesis is substantially completely covered, preferably by means of gas-phase deposition.

9. The method according to claim 7 or 8, **characterised in that** a parylene-containing coating 0.1 to 10 µm in thickness, preferably 0.4 µm to 7 µm in thickness, particularly preferably 1 µm to 5 µm in thickness is applied.

10. The method according to any one of claims 7 to 9, **characterised in that** an additional layer containing at least one pharmaceutically active substance is applied to the parylene-containing coating.

11. The method according to claim 10, **characterised in that** the additional layer containing at least one pharmaceutically active substance is applied by spraying of a solution.

## Revendications

1. Endoprothèse, notamment endoprothèse intraluminale, par exemple, un stent, avec une grille de base constituée d'au moins un matériau largement biodégradable et un revêtement (30) disposé sur le matériau biodégradable, **caractérisée en ce que** le revêtement (30) contient des parylènes, de préférence au moins surtout des parylènes, de manière particulièrement préférée du parylène C ou du parylène N, et que la grille de base est essentiellement recouverte en totalité par le revêtement (30), où l'épaisseur du revêtement (30) se situe entre 0,1 µm et 10 µm, de préférence entre 0,4 µm et 7 µm, de manière particulièrement préférée entre 1 µm et 5 µm et où le revêtement présente des régions (25, 32, 32') lesquelles ont localement une épaisseur de couche diminuée en comparaison avec les autres régions du revêtement.

2. Endoprothèse selon la revendication 1, **caractérisée en ce qu'**entre le revêtement (30) et le matériau de la grille de base une couche de promoteur d'adhésion est disposée, qui contient de préférence un ou plusieurs composés du groupe des composés magnésiens non organiques, par exemple, de l'oxyde de magnésium, du phosphate de magnésium, ou du phosphate de calcium.

3. Endoprothèse selon l'une des revendications précédentes, **caractérisée en ce que** le matériau biodégradable contient du Mg ou un alliage de Mg, de préférence du WE43, ou des polymères biodégradables, de préférence de l'acide polylactique PLLA.

4. Endoprothèse selon l'une des revendications précédentes, **caractérisée en ce que** le revêtement (30) présente en plus un ou plusieurs polymères du groupe constitué de polyesters, de préférence de polylactides, et des polypeptides.

5. Endoprothèse selon l'une des revendications précédentes, **caractérisée en ce qu'**une couche (40) avec au moins une substance pharmaceutiquement active est rapportée au moins partiellement sur le revêtement (30).

6. Endoprothèse selon la revendication 6, **caractérisée en ce que** la couche (40) avec la substance pharmaceutiquement active contient un polymère dégradable, de préférence du groupe des polyesters, en tant que support.

7. Procédé de fabrication d'une endoprothèse selon l'une des revendications 1 à 6, **caractérisé par** les étapes suivantes :
- mise à disposition de la grille de base de l'endoprothèse
- dépôt d'un revêtement contenant des parylènes sur la surface de l'endoprothèse de telle sorte que celle-ci en est essentiellement recouverte en totalité, de préférence au moyen d'un dépôt en phase vapeur, et
- réalisation d'un traitement au plasma d'oxygène.

8. Procédé de fabrication d'une endoprothèse selon l'une des revendications 1 à 6, **caractérisé par** les étapes suivantes :
- mise à disposition de la grille de base de l'endoprothèse,
- apport de points de rupture prévue dans la grille de base et
- dépôt d'un revêtement contenant des parylènes sur la surface de l'endoprothèse de telle sorte que celle-ci en est essentiellement recouverte en totalité, de préférence au moyen d'un dépôt en phase vapeur.

9. Procédé selon les revendications 7 ou 8, **caractérisé en ce qu**'un revêtement contenant des parylènes d'une épaisseur de 0,1 à 10 µm, de préférence de 0,4 µm à 7 µm, de manière particulièrement préférée de 1 µm à 5 µm est rapporté.

10. Procédé selon l'une des revendications 7 à 9, **caractérisé en ce qu'**une autre couche, qui contient au moins une substance pharmaceutiquement active est rapportée sur le revêtement contenant des parylènes.

11. Procédé selon la revendication 10, **caractérisé en ce que** l'autre couche avec au moins une substance pharmaceutiquement active est rapportée au moyen d'ne pulvérisation d'une solution.
